# EUROPÄISCHE PATENTSCHRIFT

(11) **EP 2 148 920 B1**
(45) Veröffentlichungstag und Bekanntmachung des Hinweises auf die Patenterteilung: **18.09.2019**
(21) Anmeldenummer: 08734471.9
(22) Anmeldetag: 09.04.2008
(51) Int. Cl.: C12M 1/22

(54) **BEHÄLTNIS ZUR AUFNAHME VON NÄHRMEDIEN**
RECEPTACLE FOR ACCEPTING NUTRIENT MEDIA
CONTENANT DE RÉCEPTION DE MILIEUX NUTRITIFS

(30) Priorität: 24.05.2007 DE 102007024620; 11.06.2007 DE 102007027273
(43) Veröffentlichungstag der Anmeldung: 03.02.2010
(60) Teilanmeldung: 13191654.6
(73) Patentinhaber: Merck Patent GmbH, 64293 Darmstadt (DE)
(72) Erfinder: MÜLLER, Rolf, 69221 Dossenheim (DE)
(74) Vertreter: Patent- und Rechtsanwälte Ullrich & Naumann
(86) Internationale Anmeldenummer: PCT/DE2008/000589
(87) Internationale Veröffentlichungsnummer: WO 2008/141597

(56) Entgegenhaltungen:
- EP-A- 0 171 174
- EP-A- 0 848 057
- EP-A- 1 528 100
- DE-A1- 3 128 542
- DE-A1- 3 137 495
- DE-A1- 4 301 882
- DE-C1- 19 608 009
- DE-T2- 60 009 564
- JP-A- 2004 337 079
- US-A- 3 158 553
- US-A- 3 158 553
- US-A- 5 021 351
- US-A- 5 605 836
- US-A1- 2001 024 821
- US-A1- 2004 224 382
- US-A1- 2006 240 549
- US-B2- 6 756 225

## Beschreibung

Die Erfindung betrifft ein Behältnis zur Aufnahme von Nährmedien, wobei darunter beispielsweise Nährlösungen oder Nährböden zu verstehen sind, insbesondere zur Kultivierung von Mikroorganismen, Zellkulturen, Bakterien, etc.. Das Behältnis gemäß Anspruch 1 umfasst eine Schale und einen die Schale schließenden Deckel. An dieser Stelle sei darauf hingewiesen, dass der Deckel die Schale umgreifen oder in die Schale eingreifen kann. Folglich sind die Schale und der Deckel ganz allgemein als Teile zu verstehen, die zur Bildung eines mehr oder weniger abgeschlossenen Raumes ineinander greifen. Zur Vereinfachung wird fortlaufend von Schale und Deckel gesprochen.

Die Schale und der Deckel umfassen jeweils einen in Form einer Kreisfläche ausgebildeten Boden (Schalenboden und Deckelboden) und eine vom Boden abragende, kreisringförmige Wand (Schalenwand und Deckelwand). Eine der Wände, vorzugsweise die Deckelwand, hat einen zumindest geringfügig größeren Innendurchmesser als der Außendurchmesser der anderen Wand, vorzugsweise der Schalenwand, so dass zum Schließen der Schale die eine Wand über die andere (oder umgekehrt) stülpbar ist.

Behältnisse der in Rede stehenden Art sind unter der Bezeichnung "Petrischale" bekannt. Darunter versteht man eine flache, runde, meist durchsichtige Glas- oder Kunststoffschale mit übergreifendem Deckel. Eine solche Schale kommt üblicherweise in der Biologie oder Chemie zum Einsatz. Sie dient zur Kultivierung von Mikroorganismen und wird zum Anlegen von Zellkulturen genutzt.

Die DE4301882 offenbart einen Kulturbemalter,mit einem Behälterunterteil und einem Deckel und in Umfangrichtung verlaufende Rippen, die auf der außenliegenden Mantelfläche des Behälterunterteils vorgesehen sind. Mit dem Behältnis aus DE4301882 ist keine unterschiedliche Klemm- und Dichtwirkung zu erzielen. Die US3158553 offenbart eine Petrischale, die als geschlossene Einheit oder als offene Einheit zu verwenden ist. Die Schale weist Nasen auf, die seitlich von Rand weg sich erstrecken. Der Deckel verfügt über keilförmige Bereiche, die auf die Nasen des Deckels auflegbar sind. Weder eine Klemm- oder Dichtwirkung noch ein unbeabsichtigtes Lösen von Deckel und Schale wird durch die Ausführung in US3158553 erreicht.Die EP1528100 offenbart eine verschließbare Petrischale bei der ein versehentliches Öffnen durch eine Verschlussvorrichtung verhindert wird, wobei radial verlaufende Erhebungen unter die Eingriffsmittel durch Verdrehen des Deckels einrasten. Diese Eingriffsmittel sind jeweils paarweise angeordnet.

In Bezug auf das gattungsbildende Behältnis sei lediglich beispielhaft auf die DE 44 06 725 A1 verwiesen, aus der ein Behältnis im Sinne einer Petrischale bekannt ist. Ein Deckel dient zum Verschluss des Behältnisses. Regelmäßig wird der Deckel über die Schale bzw. über die Schalenwand gestülpt. Die Handhabung eines insoweit geschlossenen Behältnisses ist jedoch problematisch, da beim Ergreifen des Deckels ein Lösen von Schale und Deckel nicht zu vermeiden ist. Verunreinigungen gelangen so ins Innere des Behältnisses oder angelegte Kulturen können nach außerhalb des Behältnisses gelangen. Es besteht Kontaminationsgefahr.

Aus der Praxis ist bereits bekannt, durch eine geeignete Ausgestaltung und Dimensionierung von Schale und Deckel zwischen der Schale und dem Deckel eine Klemmwirkung hervorzurufen. Wollte man dabei eine sichere Verbindung zwischen der Schale und dem Deckel generieren, müsste man eine Art Presspassung realisieren, die ein Lösen des Deckels von der Schale erschwert, wenn nicht sogar unmöglich macht. Folglich ist eine solche Lösung zum sicheren Verschleißen und Öffnen des Behältnisses nur bedingt geeignet.

Der vorliegenden Erfindung liegt nun die Aufgabe zugrunde, ein Behältnis zur Aufnahme von Nährmedien, insbesondere zum Kultivieren von Mikroorganismen, Zellkulturen bzw. Organismen, derart auszugestalten und weiterzubilden, dass eine sichere Handhabung insbesondere bei verschlossenem Behältnis möglich. Die Gefahr eines unbeabsichtigten Lösens des Deckels von der Schale soll weitestgehend vermieden sein, wobei ein gewolltes Lösen des Deckels von der Schale ohne große Kraftaufwendung möglich sein muss.

Erfindungsgemäß ist die voranstehende Aufgabe durch die Merkmale des Patentanspruches 1 gelöst. Danach ist das hier in Rede stehende Behältnis dadurch gekennzeichnet, dass der Schale und dem Deckel Eingriffsmittel zugeordnet sind, die bei gegenseitigem Eingriff ein unbeabsichtigtes Lösen des Deckels von der Schale verhindern.

In erfindungsgemäßer Weise ist erkannt worden, dass die aus der Praxis bekannte Klemmwirkung zwischen Schale und Deckel einerseits unzureichend ist und andererseits die Gefahr in sich birgt, dass sich der Deckel von der Schale aufgrund einer besonders starken Klemmwirkung nicht lösen lässt. So ist man in erfindungsgemäßer Weise von einer konventionellen Verbindung zwischen Schale und Deckel aufgrund einer Klemmwirkung, nämlich aufgrund einer Presspassung, abgegangen, und hat eine Verbindung durch Eingriffsmittel gewählt, wobei diese Eingriffsmittel sowohl der Schale als auch dem Deckel zugeordnet sind. Die Eingriffsmittel sind derart ausgebildet bzw. konstruiert, dass bei gegenseitigem Eingriff der Eingriffsmittel ein unbeabsichtigtes Lösen des Deckels von der Schale wirksam verhindert ist. Bringt man die Eingriffsmittel außer Eingriff, lässt sich der Deckel mühelos von der Schale entfernen.

In Bezug auf eine konstruktiv einfache Ausgestaltung des sind die Eingriffsmittel integrale Bestandteile der Schale einerseits und des Deckels andererseits. Dabei können die Eingriffsmittel der Deckelwand und der Schalenwand zugeordnet sein. Auch ist es denkbar, dass die Eingriffsmittel einerseits der Deckelwand und andererseits einem umlaufenden äußeren Randbereich der Schale zugeordnet sind. Wesentlich ist dabei lediglich, dass sowohl der Schale als auch dem Deckel Eingriffsmittel zugeordnet sind, die beim Schließen der Schale durch den Deckel in gegenseitigen Eingriff bringbar sind, so dass ein unbeabsichtigtes Lösen des Deckels von der Schale ausgeschlossen ist.

In weiter vorteilhafter Weise dient der äußere Randbereich der Schale, der aufgrund der Anordnung bzw. Ausbildung der Schalenwand außerhalb der Schale liegt, zur Anlage für die Deckelwand, und zwar dann, wenn die Deckelwand über die Schalenwand greift.

Entsprechend ist es von Vorteil, wenn die Schalenwand vom äußeren Randbereich nach innen versetzt ausgebildet ist und wenn der außerhalb der Schalenwand liegende Randbereich, vorzugsweise ganz außen, die Eingriffsmittel trägt. An dieser Stelle sei angemerkt, dass es hier ausschließlich um den gegenseitigen Eingriff der Eingriffsmittel geht, die sowohl der Schale als auch dem Deckel zugeordnet sind. Alternativ ist eine Ausgestaltung von Schale und Deckel der Gestalt möglich, dass die Schalenwand die Deckelwand übergreift, so dass der Deckel in die Schale, d. h. in den Bereich innerhalb der Schalenwand, einsetzbar ist. Auch im Rahmen einer solchen Ausgestaltung ist ein gegenseitiges in Eingriff bringen der Eingriffsmittel möglich, so dass auch insoweit die erfindungsgemäße Lehre anwendbar ist.

Die Eingriffsmittel des Deckels am freien Rand oder nahe des freien Randes der Deckelwand ausgebildet. Die Eingriffsmittel des Deckels sind als orthogonal von der Deckelwand nach außen abragende Flansche ausgeführt. Diese Eingriffsmittel dienen zum Eingriff in entsprechende Eingriffsmittel an der Schale, wobei die Eingriffsmittel des Deckels in die Eingriffsmittel der Schale einschiebbar sind.

Die Eingriffsmittel der Schale sind entsprechend der Ausgestaltung der Eingriffsmittel des Deckels ausgebildet, nämlich um die Eingriffsmittel des Deckels formschlüssig und/oder kraftschlüssig aufnehmen zu können. Die Eingriffsmittel der Schale sind flanschartig zum zumindest teilweisen Um- und/oder Ein- und/oder Übergreifen der Eingriffsmittel des Deckels ausgeführt sind. Dabei handelt es sich um Eingriffsmittel gemäß Anspruch 1. oder um eine sonstige Erhöhung zum klemmenden Eingriff handeln.

Die Eingriffsmittel des Deckels sind hälftig von beiden Seiten her mit unterschiedlicher Klemm- und Dichtwirkung einschiebbar und haben unterschiedlich hohe Keilflächen oder Erhebungen so, so dass die Eingriffsmittel des Deckels von beiden Seiten her mit unterschiedlicher Klemm- und Dichtwirkung einschiebbar sind. Im Rahmen einer solchen Ausgestaltung ist es möglich, den Deckel bzw. die Eingriffsmittel des Deckels jeweils links oder rechts von den Eingriffsmitteln der Schale zu positionieren und durch Drehen gegenüber der Schale die Eingriffsmittel des Deckels in die Eingriffsmittel der Schale - von links oder von rechts her - einzuschieben. Aufgrund der unterschiedlichen Keilflächen oder unterschiedlicher Erhöhung entsteht von der einen Seite her ein fester, abdichtender Verschluss der Schale und von der anderen Seite her Verschluss mit definiertem Schlitz bzw. Spalt zwischen der Schale und dem Deckel zum Gasaustausch. Somit lassen sich im Behältnis unterschiedliche Bedingungen für die jeweiligen Kulturen schaffen.

In Bezug auf ein sicheres Schließen der Schale ist es erforderlich, dass mindestens zwei vorzugsweise äquidistant angeordnete Eingriffsmittel (7, 8) vorgesehen sind. Eine kippsichere Anordnung des Deckels gegenüber der Schals und somit ein sicheres Schließen wird durch die Vorkehrung von drei äquidistant angeordneten Eingriffsmitteln (7, 8) bewerkstelligt, so dass der Deckel entlang seines Umfanges gleichmäßig zur Schale anordenbar ist.

In weiter vorteilhafter Weise lässt sich die Verbindung bzw. Positionierung des Deckels zur Schale dadurch begünstigen, dass die Schalenwand auf der Außenseite und/oder die Deckelwand auf der Innenseite Abstandhalter zur Beabstandung und ggf. zur Klemmung zwischen Deckelwand und Schalewand hat, so dass eine Belüftung der Schale dann jedenfalls gewährleistet ist, wenn sich der Deckel in der offenen, d. h. einen Spalt gegenüber der Schale bildenden Position befindet. Die geschlossene Position, ohne Vorkehrung eines Spalts zwischen der Schale und dem Deckel, wird dann erreicht, wenn die Deckelwand komplett an der Schale bzw. an den Randbereich der Schale gedrückt wird, nämlich durch entsprechendes Zusammenwirken zwischen den Eingriffsmitteln der Schale und des Deckels.

Im Konkreten können die Abstandhalter als sich vom Boden orthogonal erstreckende Stege ausgeführt sein. Entlang des Umfangs der Schalenwand lassen sich mehrere Abstandhalter entsprechend den voranstehenden Ausführungen anbringen, wobei es von ganz besonderem Vorteil ist, wenn gegenüber eines jeden Eingriffsmittels auch ein Abstandhalter angeordnet ist, so dass die Deckelwand dazwischen, d. h. im Bereich zwischen einem Eingriffsmittel und einem Abstandhalter, eingeführt bzw. eingesteckt werden kann. Diese Maßnahme begünstigt ein sicheres Positionieren des Deckels gegenüber der Schale.

Die Abstandhalter sind vorzugsweise äquidistant in der Schalenwand und/oder in der Deckelwand ausgebildet, wobei die Vorkehrung hinreichend vieler Abstandhalter eine gleichmäßige Beabstandung zwischen der Deckelwand und Schalenwand gewährleistet.

Die Handhabung des erfindungsgemäßen Behältnisses wird dadurch begünstigt, dass auf der der Schalenwand abgewanden Seite des Schalenbodens eine vorzugsweise am äußeren Ende des Randbereichs in entgegengesetzte Richtung abragende Basis in Form einer kreisringförmigen Wand ausgebildet ist, die zum Positionieren und ggf. Stapeln der Schale dient. Diese Wand bildet eine Art Podest für die eigentliche Schale und vereinfacht nicht nur die Handhabung sondern auch die Lagerung im leeren oder gefüllten Zustand.

In weiter vorteilhafter Weise ist es denkbar, dass sowohl die Schale als auch der Deckel, vorzugsweise mit entsprechender Zuordnung, mit einer vorzugsweise maschinenlesbaren Codierung (z. B. in Form eines Data Matrix Codes) versehen sind. Mit einer solchen Codierung lassen sich die angelegten Kulturen eindeutig zuordnen bzw. identifizieren.

Schließlich sei angemerkt, dass die Schale und der Deckel aus Glas oder aus vorzugsweise durchsichtigem Kunststoff hergestellt sein können. Im Falle einer zu bevorzugenden Ausgestaltung aus Kunststoff ist es denkbar, dass die Schale und der Deckel spritzgusstechnisch hergestellt sind. Eine spritzgusstechnische Fertigung hat den enormen Vorteil, dass sich sämtliche Bestandteile sowohl des Deckels als auch der Schale als integrale Bestandteile ausbilden lassen, was die Herstellkosten ganz erheblich reduziert.

Die Lehre der vorliegenden Erfindung in vorteilhafter Weise auszugestalten und weiterzubilden ist auf die dem Patentanspruch 1 nachgeordneten Patentansprüchen zu verweisen.
- Fig. 1: in einer schematischen Draufsicht ein Ausführungsbeispiel einer Schale eines erfindungsgemäßen Behältnisses,
- Fig. 2a: den Gegenstand aus Fig. 1, teilweise und vergrößert, im Schnitt entlang der Linie A-A,
- Fig. 2b: den Gegenstand aus Fig. 1 im Schnitt entlang der Linie C-C,
- Fig. 3a: in einer schematischen Draufsicht das Behältnis mit Schale und Deckel, wobei sich der Deckel in der offenen Position befindet,
- Fig. 3b: den Gegenstand aus Fig. 3a im Schnitt entlang der Linie D-D,
- Fig. 4a: in einer schematischen Draufsicht das Behältnis mit Schale und Deckel, wobei sich der Deckel in der geschlossenen Position befindet,
- Fig. 4b: den Gegenstand aus Fig. 4a im Schnitt entlang der Linie E-E,
- Fig. 5a: in einer schematischen Draufsicht den Deckel des erfindungsgemäßen Behältnisses und
- Fig. 5b: den Gegenstand aus Fig. 5a im Schnitt entlang der Linie B-B.

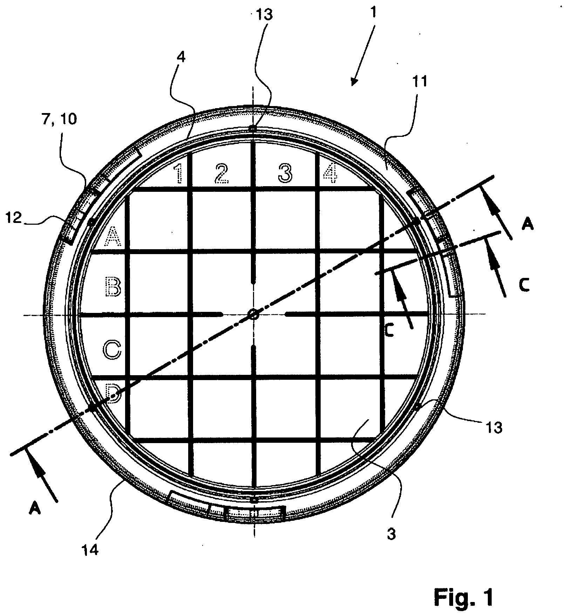

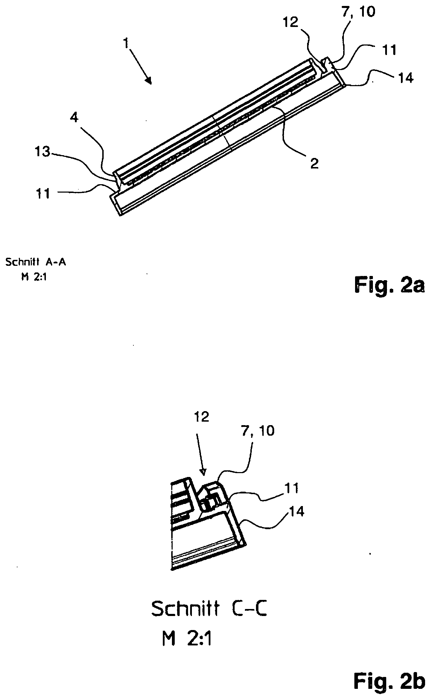

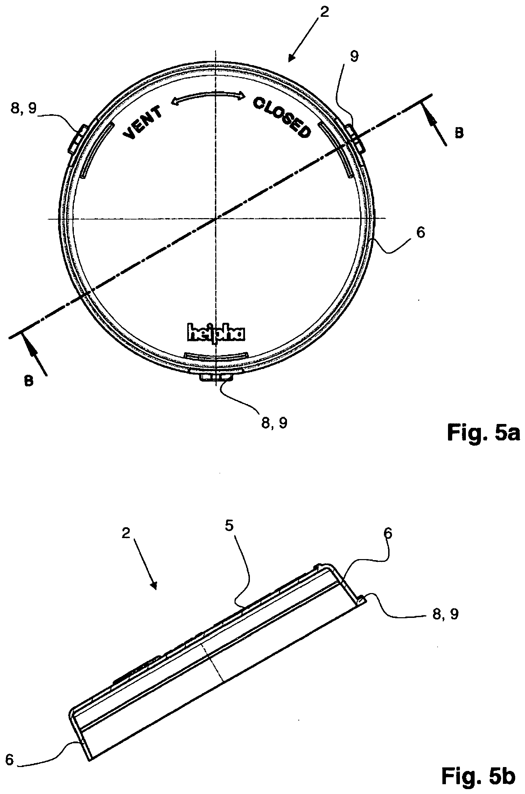

Die Fig. 1 bis 5 zeigen in schematischen Ansichten ein Ausführungsbeispiel eines erfindungsgemäßen Behältnisses zur Aufnahme von Nährmedien, insbesondere zur Aufnahme von Zellkulturen, Bakterien, etc. jedweder Art. Im Konkreten handelt es sich dabei um besondere Ausbildungen einer Petrischale.

Das Behältnis umfasst eine Schale 1 und einen Deckel 2. Der Deckel 2 dient zum Schließen der Schale 1.

Die Schale 1 umfasst einen in Form einer Kreisfläche ausgebildeten Schalenboden 3 und eine vom Schalenboden 3 abragende, kreisringförmige Schalenwand 4.

Der Deckel 2 umfasst entsprechend einen Deckelboden 5 und eine Deckelwand 6.

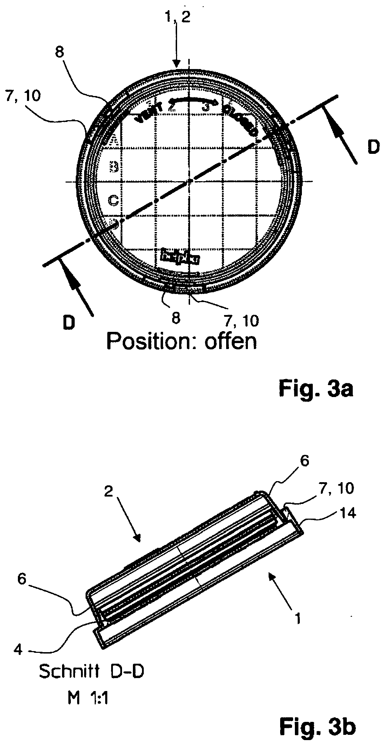

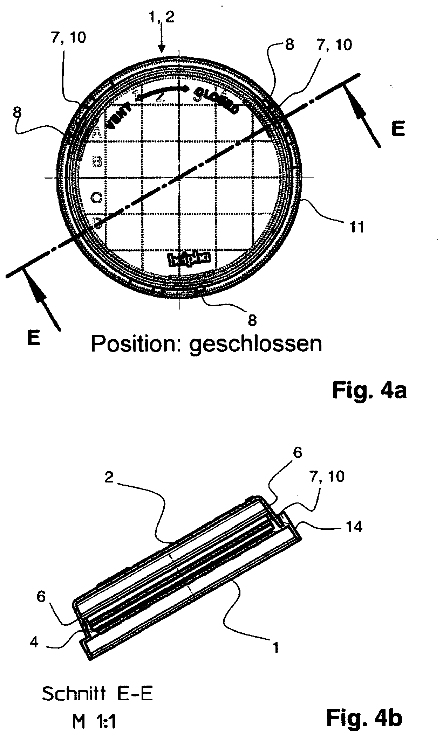

Die Fig. 3 und 4 lassen erkennen, dass die Deckelwand 6 einen zumindest geringfügig größeren Innendurchmesser als der Außendurchmesser der Schalenwand 4 hat. Dadurch ist gewährleistet, dass sich der Deckel 2 mit seiner Deckelwand 6 über die Schalenwand 4 der Schale 1 stülpen lässt, um nämlich die Schale 1 zu schließen. Letztendlich geht es darum, dass die beiden Teile - Schale und 1 und Deckel 2 - gemeinsam einen Raum bilden, wobei das eine Teil über das andere bzw. in das andere greift.

Erfindungsgemäß sind der Schale 1 und dem Deckel 2 Eingriffsmittel 7, 8 zugeordnet, die bei gegenseitigem Eingriff ein unbeabsichtigtes Lösen von Schale 1 und Deckel 2 verhindern.

Die Schale weist komplementär ausgebildete Flansche 10 auf, die klammerartig ausgebildet sind. Die Flanschen 10 erstrecken sich von einem Randbereich 11 des Schalenbodens aus orthogonal und haben eine für das Halten bzw. die Klemmwirkung verantwortliche Schulter 12. Die Rastnase 9 des Deckels 2 lässt sich von beiden Seiten eines Flansches 10 her in den Bereich unter die Schulter 12 einschieben, wobei dort - jeweils rechts und links - unterschiedlich ausgebildete Keilflächen oder sonstige Erhebungen vorgesehen sind. Diese konstruktive Maßnahme führt dazu, dass sich je nach Einschieben von rechts oder von links her ein zumindest geringfügig beabstandetes oder dichtes Anbringen des Deckels 2 generieren lässt, wodurch innerhalb des Behältnisses eine offene (vgl. Fig. 3) oder geschlossene (vgl. Fig. 4) Situation schaffbar ist.

Die Fig. zeigen des Weiteren, dass die Schalenwand 4 auf deren Außenseite Abstandhalter 13 aufweist, die entlang des Umfanges der Schalenwand 4 ausgebildet sind. Diese Abstandhalter 13 schaffen eine Art Ringspalt zwischen der Innenseite der Deckelwand 6 und der Außenseite der Schalenwand 4 (vgl. Fig. 3 und 4), so dass bei nicht ganz anliegendem Deckel 2 auf dem Randbereich 11 eine zumindest geringfügige Belüftung bzw. Hinterlüftung realisierbar ist. Liegt der freie Rand der Deckelwand 6 komplett am Randbereich 11 an, wird nämlich durch die Eingriffsmittel 7, 8 eine Klemmwirkung hervorgerufen, ist eine geschlossene Situation innerhalb des Behältnisses geschaffen (Fig. 4).

Des Weiteren zeigen die Fig., dass die Schale 1 eine integrale Basis 14 in Form einer kreisringförmigen Wand aufweist, die vom Randbereich 11 aus, auf der der Schalenwand 4 abgewanden Seite des Schalenbodens 3, abragt. Die Basis 14 dient einerseits zum sicheren Positionieren und andererseits zum Stapeln des Behältnisses.

Schließlich sei angemerkt, dass das voranstehend erörterte Ausführungsbeispiel nebst Fig. lediglich der beispielhaften Erörterung der beanspruchten Lehre dient, dies jedoch nicht auf die Ausführungsbeispiele einschränken.

## Patentansprüche

1. Behältnis zur Aufnahme von Nährmedien, insbesondere zur Kultivierung von Mikroorganismen, Zellkulturen, Bakterien, mit einer Schale (1) und einem die Schale (1) schließenden Deckel (2), wobei die Schale (1) einen in Form einer Kreisfläche ausgebildeten Schalenboden (3) und eine vom Schalenboden (3) abragende, kreisringförmige Schalenwand (4) umfasst und wobei der Deckel (2) einen in Form einer Kreisfläche ausgebildeten Deckelboden (5) und eine vom Deckelboden (5) abragende, kreisringförmige Deckelwand (6) umfasst, wobei die Deckelwand (6) einen größeren Innendurchmesser als der Außendurchmesser der Schalenwand (4) hat oder die Schalenwand (4) einen größeren Innendurchmesser als der Außendurchmesser der Deckelwand (6) hat, so dass zum Schließen der Schale (1) die Deckelwand (6) über die Schalenwand (4) oder die Schalenwand (4) über die Deckelwand (6) stülpbar ist, wobei der Schale (1) und dem Deckel (2) Eingriffsmittel (7, 8) zugeordnet sind, die bei gegenseitigem Eingriff ein unbeabsichtigtes Lösen von Schale (1) und Deckel (2) verhindern, wobei die Eingriffsmittel (7) der Schale (1) klammerartige Flansche (10) zum Um- oder Eingreifen der Eingriffsmittel (8) des Deckels (2) ausgeführt sind und wobei sich die Flansche (10) von einem Randbereich (11) des Schalenbodens aus orthogonal erstrecken und eine für das Halten oder die Klemmwirkung verantwortliche Schulter (12) aufweisen,
**dadurch gekennzeichnet, dass** die Eingriffsmittel (8) des Deckels (2), in Umfangsrichtung des Deckels (2) gesehen hälftig von beiden Seiten her, unterschiedlich hohe Keilflächen oder Erhöhungen haben, so dass die Eingriffsmittel (8) des Deckels (2) in Umfangsrichtung der Deckels (2) gesehen von beiden Seiten her mit unterschiedlicher Klemm- und Dichtwirkung in die Eingriffsmittel (7) der Schale (1), nämlich in den Bereich unter die Schulter (12) einschiebbar sind.

2. Behältnis nach Anspruch 1, **dadurch gekennzeichnet, dass** die Eingriffsmittel (7, 8) integrale Bestandteile der Schale (1) und des Deckels (2) sind.

3. Behältnis nach Anspruch 1 oder 2, **dadurch gekennzeichnet, dass** die Eingriffsmittel (7, 8) der Deckelwand (6) und der Schalenwand (4) zugeordnet sind.

4. Behältnis nach Anspruch 1 oder 2, **dadurch gekennzeichnet, dass** die Eingriffsmittel (7, 8) der Deckelwand (6) und einem umlaufenden äußeren Randbereich (11) der Schale (1) zugeordnet sind, wobei der äußere Randbereich (11) der Schale (1) zur Anlage für die Deckelwand (6) dient.

5. Behältnis nach Anspruch 4, **dadurch gekennzeichnet, dass** die Schalenwand (4) vom äußeren Randbereich (11) nach innen versetzt ausgebildet ist und dass der außerhalb der Schalenwand (4) liegende Randbereich (11), vorzugsweise ganz außen, die Eingriffsmittel (7) trägt.

6. Behältnis nach einem der Ansprüche 1 bis 5, **dadurch gekennzeichnet, dass** die Eingriffsmittel (8) des Deckels (2) mindestens eine Rastposition umfassen können.

7. Behältnis nach einem der Ansprüche 1 bis 6, **dadurch gekennzeichnet, dass** die Eingriffsmittel (7) der Schale (1) mindestens eine Keilfläche oder Erhöhung zum klemmenden Eingriff mit den Eingriffsmitteln (8) des Deckels (2) haben.

8. Behältnis nach einem der Ansprüche 1 bis 7, **dadurch gekennzeichnet, dass** die Eingriffsmittel (7) der Schale (1) derart ausgebildet sind, dass die Eingriffsmittel (8) des Deckels (2) durch Drehen des Deckels (2) gegenüber der Schale (1) in Eingriff bringbar sind.

9. Behältnis nach einem der Ansprüche 1 bis 8, **dadurch gekennzeichnet, dass** der beidseitige Eingriff derart ausgelegt ist, dass von einer Seite her ein festes und/oder abdichtendes Verschließen und von der anderen Seite her ein Verschließen mit einem definierten Schlitz oder Spalt zum Gasaustausch möglich ist.

10. Behältnis nach einem der Ansprüche 1 bis 9, **dadurch gekennzeichnet, dass** der Schale (1) und dem Deckel (2) mindestens zwei, vorzugsweise drei äquidistant angeordnete Eingriffsmittel (7, 8) zugeordnet sind.

11. Behältnis nach einem der Ansprüche 1 bis 10, **dadurch gekennzeichnet, dass** die Schalenwand (4) auf der Außenseite und/oder die Deckelwand (6) auf der Innenseite Abstandhalter zur Beabstandung zwischen Deckelwand (6) und Schalenwand (4) hat, wobei die Abstandhalter (13) als sich vom Boden orthogonal erstreckende Stege ausgeführt sind und/oder wobei gegenüber eines Eingriffsmittels (7, 8) ein Abstandhalter (13) ausgebildet sein kann.

12. Behältnis nach Anspruch 11, **dadurch gekennzeichnet, dass** die Abstandhalter (13) vorzugsweise äquidistant in der Schalenwand (4) und/oder in der Deckelwand (6) ausgebildet sind.

13. Behältnis nach einem der Ansprüche 1 bis 12, **dadurch gekennzeichnet, dass** auf der der Schalenwand (4) abgewandten Seite des Schalenbodens (3) eine vorzugsweise am äußeren Ende des Randbereichs (11) in entgegen gesetzte Richtung abragende Basis (14) in Form einer kreisringförmigen Wand ausgebildet ist, die zum Positionieren der Schale (1) dient.

14. Behältnis nach einem der Ansprüche 1 bis 13, **dadurch gekennzeichnet, dass** die Schale (1) und/oder der Deckel (2) mit einer vorzugsweise maschinenlesbaren Codierung versehen sind.

15. Behältnis nach einem der Ansprüche 1 bis 14, **dadurch gekennzeichnet, dass** die Schale (1) und der Deckel (2) aus vorzugsweise durchsichtigem Kunststoff hergestellt sind, wobei die Schale (1) und der Deckel (2) spritzgusstechnisch hergestellt sind.

## Claims

1. Container for receiving nutrient media, in particular for culturing microorganisms, cell cultures and bacteria, having a dish (1) and a lid (2) which closes the dish (1), the dish (1) comprising a dish base (3) in the form of a circular surface, and an annular dish wall (4) projecting from the dish base (3), the lid (2) comprising a lid base (5) in the form of a circular surface, and an annular lid wall (6) projecting from the lid base (5), the lid wall (6) having a larger inner diameter than the outer diameter of the dish wall (4), or the dish wall (4) having a larger inner diameter than the outer diameter of the lid wall (6), such that in order to close the dish (1) the lid wall (6) can be slipped over the dish wall (4) or the dish wall (4) can be slipped over the lid wall (6), engagement means (7, 8) being associated with the dish (1) and the lid (2), which engagement means, when mutually engaged, prevent an unintentional disengagement of the dish (1) and the lid (2), the engagement means (7) of the dish (1) being designed as clamp-like flanges (10) for encompassing or engaging the engagement means (8) of the lid (2), and the flanges (10) extending orthogonally from an edge region (11) of the dish base and having a shoulder (12) which is responsible for the holding or the clamping effect, **characterised in that** the engagement means (8) of the lid (2), seen equally from both sides in the circumferential direction of the lid (2), have wedge surfaces or elevations of different heights, such that the engagement means (8) of the lid (2), seen from both sides in the circumferential direction of the lid (2), can be inserted with a different clamping and sealing effect into the engagement means (7) of the dish (1), namely into the region under the shoulder (12).

2. Container according to claim 1, **characterised in that** the engagement means (7, 8) are integral components of the dish (1) and the lid (2).

3. Container according to either claim 1 or claim 2, **characterised in that** the engagement means (7, 8) are associated with the lid wall (6) and the dish wall (4).

4. Container according to either claim 1 or claim 2, **characterised in that** the engagement means (7, 8) are associated with the lid wall (6) and with a circumferential outer edge region (11) of the dish (1), the outer edge region (11) of the dish (1) being used as an abutment surface for the lid wall (6).

5. Container according to claim 4, **characterised in that** the dish wall (4) is offset inwardly from the outer edge region (11), and **in that** the edge region (11) positioned outside the disc wall (4), preferably completely outside, carries the engagement means (7).

6. Container according to any one of claims 1 to 5, **characterised in that** the engagement means (8) of the lid (2) can comprise at least one locking position.

7. Container according to any one of claims 1 to 6, **characterised in that** the engagement means (7) of the dish (1) have at least one wedge surface or elevation for a clamping engagement with the engagement means (8) of the lid (2).

8. Container according to any one of claims 1 to 7, **characterised in that** the engagement means (7) of the dish (1) are designed such that the engagement means (8) of the lid (2) can be brought into engagement with the dish (1) by turning the cover (2).

9. Container according to any one of claims 1 to 8, **characterised in that** the two-sided engagement is designed such that from one side a firm and/or sealing closure is possible and from the other side a closure which has a defined slot or gap for gas exchange is possible.

10. Container according to any one of claims 1 to 9, **characterised in that** at least two, preferably three equidistantly arranged engagement means (7, 8) are associated with the dish (1) and the lid (2).

11. Container according to any one of claims 1 to 10, **characterised in that** the dish wall (4) on the outer face, and/or the lid wall (6) on the inner face, has spacers for spacing between the lid wall (6) and dish wall (4), the spacers (13) being designed as ridges which extend orthogonally from the base, and/or it being possible for a spacer (13) to be formed opposite an engagement means (7, 8).

12. Container according to claim 11, **characterised in that** the spacers (13) are preferably formed equidistantly in the dish wall (4) and/or in the lid wall (6).

13. Container according to any one of claims 1 to 12, **characterised in that** a foot (14) in the form of an annular wall is formed on the side of the dish base (3) that faces away from the dish wall (4), preferably at the outer end of the edge region (11), which foot projects in the opposite direction and is used to position the dish (1).

14. Container according to any one of claims 1 to 13, **characterised in that** the dish (1) and/or the lid (2) are provided with a preferably machine-readable coding.

15. Container according to any one of claims 1 to 14, **characterised in that** the dish (1) and the lid (2) are made of preferably transparent plastics material, the dish (1) and the lid (2) being produced by means of injection moulding.

## Revendications

1. Récipient pour le stockage de milieux nutritifs, plus particulièrement pour la culture de micro-organismes, cultures cellulaires, bactéries, avec une coque (1) et un couvercle (2) fermant la coque (1), la coque (1) comprenant un fond de coque (3) conçu sous la forme d'une surface circulaire et une paroi de coque (4) en forme d'anneau circulaire partant du fond de coque (3) et le couvercle (2) comprenant un fond de couvercle (5) conçu sous la forme d'une surface circulaire et une paroi de couvercle (6) en forme d'anneau circulaire, partant du fond de couvercle (5), la paroi de couvercle (6) présentant un diamètre intérieur supérieur au diamètre extérieur de la paroi de coque (4) ou la paroi de coque (4) présentant un diamètre intérieur supérieur au diamètre extérieur de la paroi de couvercle (6), de façon à ce que, pour la fermeture de la coque (1), la paroi de couvercle (6) puisse être rabattue par-dessus la paroi de coque (4) ou la paroi de coque (4) puisse être rabattue par-dessus la paroi de couvercle (6), la coque (1) et le couvercle (2) étant munies de moyens d'emboîtement (7, 8) qui, lors d'un emboîtement mutuel, empêchent un détachement par inadvertance de la coque (1) et du couvercle (2), les moyens d'emboîtement (7) de la coque (1) étant conçus comme des brides en formes d'agrafes (10) pour s'emboîter avec les moyens d'emboîtement (8) du couvercle (2) et les brides (10) s'étendant à partir d'un bord (11) du fond de coque de manière orthogonale et comprenant un épaulement (12) responsable du maintien ou de l'effet de serrage,
**caractérisé en ce que** les moyens d'emboîtement (8) du couvercle (2), vus dans la direction de la circonférence du couvercle, présentent, à moitié à partir des deux côtés, des surfaces cunéiformes ou des bossages de différentes hauteurs, de façon à ce que les moyens d'emboîtement (8) du couvercle (2), vus dans la direction de la circonférence du couvercle (2) puissent être insérés, des deux côtés, avec un effet de serrage et d'étanchéité différent, dans les moyens d'emboîtement (7) de la coque (1), à savoir dans la partie sous l'épaulement (12).

2. Récipient selon la revendication 1, **caractérisé en ce que** les moyens d'emboîtement (7, 8) font partie intégrante de la coque (1) et du couvercle (2).

3. Récipient selon la revendication 1 ou 2, **caractérisé en ce que** les moyens d'emboîtement (7, 8) de la paroi de couvercle (6) et de la paroi de coque (4) correspondent.

4. Récipient selon la revendication 1 ou 2, **caractérisé en ce que** les moyens d'emboîtement (7, 8) de la paroi de couvercle (6) et un bord externe circulaire (11) de la coque (1) correspondent, le bord externe (11) de la coque (1) servant d'appui pour la paroi de couvercle (6).

5. Récipient selon la revendication 4, **caractérisé en ce que** la paroi de coque (4) est conçue de manière décalée vers l'intérieur par rapport au bord externe (11) et **en ce que** le bord (11) se trouvant à l'extérieur de la paroi de coque (4), de préférence entièrement à l'extérieur, supporte les moyens d'emboîtement (7).

6. Récipient selon l'une des revendications 1 à 5, **caractérisé en ce que** les moyens d'emboîtement (8) du couvercle (2) peuvent comprendre au moins une position d'encliquetage.

7. Récipient selon l'une des revendications 1 à 6, **caractérisé en ce que** les moyens d'emboîtement (7) de la coque (1) comprennent au moins une surface cunéiforme ou un bossage pour l'emboîtement de serrage avec les moyens d'emboîtement (8) du couvercle (2).

8. Récipient selon l'une des revendications 1 à 7, **caractérisé en ce que** les moyens d'emboîtement (7) de la coque (1)sont conçus de façon à ce que les moyens d'emboîtement (8) du couvercle (2) puissent être emboîtés par la rotation du couvercle (2) par rapport à la coque (1).

9. Récipient selon l'une des revendications 1 à 8, **caractérisé en ce que** l'emboîtement mutuel est conçu de façon à ce que, d'un côté, une fermeture fixe et/ou étanche et de l'autre côté, une fermeture avec une fente ou un interstice défini pour un échange de gaz soit possible.

10. Récipient selon l'une des revendications 1 à 9, **caractérisé en ce que**, à la coque (1) et au couvercle (2) correspondent au moins deux, de préférence trois moyens d'emboîtement (7, 8) disposés de manière équidistante.

11. Récipient selon l'une des revendications 1 à 10, **caractérisé en ce que** la paroi de coque (4) comprend, sur le côté extérieur et/ou la paroi de couvercle (6) comprend, sur le côté intérieur, des entretoises pour l'écartement entre la paroi de couvercle (6) et la paroi de coque (4), les entretoises (13) pouvant être réalisées comme des nervures s'étendant de manière orthogonale à partir du fond et/ou, en face d'un moyen d'emboîtement (7, 8), une entretoise (13) pouvant être prévue.

12. Récipient selon la revendication 11, **caractérisé en ce que** les entretoises (13) sont conçues de préférence de manière équidistante dans la paroi de coque (4) et/ou dans la paroi de couvercle (6).

13. Récipient selon l'une des revendications 1 à 12, **caractérisé en ce que**, sur le côté du fond de coque (3) opposé à la paroi de coque (4), est réalisée, de préférence à l'extrémité externe du bord (11), une base (14) partant dans la direction opposée, sous la forme d'une paroi annulaire qui permet le positionnement de la coque (1).

14. Récipient selon l'une des revendications 1 à 13, **caractérisé en ce que** la coque (1) et/ou le couvercle (2) sont munis d'un codage lisible de préférence à l'aide d'une machine.

15. Récipient selon l'une des revendications 1 à 14, **caractérisé en ce que** la coque (1) et le couvercle (2) sont constitués de préférence d'une matière plastique transparente, la coque (1) et le couvercle (2) étant fabriqués par moulage par injection.
